# EUROPEAN PATENT APPLICATION

(11) **EP 4 650 601 A1**
(43) Date of publication of application: **19.11.2025**
(21) Application number: 25176639.0
(22) Date of filing: 15.05.2025
(51) Int. Cl.: F04B 11/00, B01D 63/00, F04B 43/12

(54) **LIQUID FEEDING ASSISTANCE DEVICE, CELL PROCESSING APPARATUS, AND LIQUID FEEDING ASSISTANCE METHOD**

(30) Priority: 16.05.2024 JP 2024080414
(71) Applicant: FUJIFILM Corporation, Tokyo 106-8620 (JP)
(72) Inventor: NAKAI, Yuki, Ashigarakami-gun, Kanagawa, 258-8577 (JP)
(74) Representative: Hoffmann Eitle

(57) **Abstract**

There are provided a liquid feeding assistance device, a cell processing apparatus, and a liquid feeding assistance method that can suppress the pulsation of liquid flowing in a flow passage.

A liquid feeding assistance device includes a tube that is expandable and contractible depending on a flow velocity of liquid flowing in the tube, and a gripping member that grips the tube with a relatively small force during expansion of the tube and grips the tube with a relatively large force during contraction of the tube.

## Description

### BACKGROUND OF THE INVENTION

### 1. Field of the Invention

A disclosed technology relates to a liquid feeding assistance device, a cell processing apparatus, and a liquid feeding assistance method.

### 2. Description of the Related Art

The following technologies are known as technologies for suppressing the pulsation of liquid flowing in a flow passage. For example, JP1988-275889A (JP-S63-275889A) discloses a pulsation prevention device including a flexible tube and a member that acts as an elastic body elastically restricting a cross-sectional area of the tube to be less than a maximum cross-sectional area.

JP2000-259253A discloses a pressure fluctuation prevention device including a flexible tube, an operation member that presses and deforms the flexible tube, and an elastic pressure mechanism that elastically presses the operation member.

### SUMMARY OF THE INVENTION

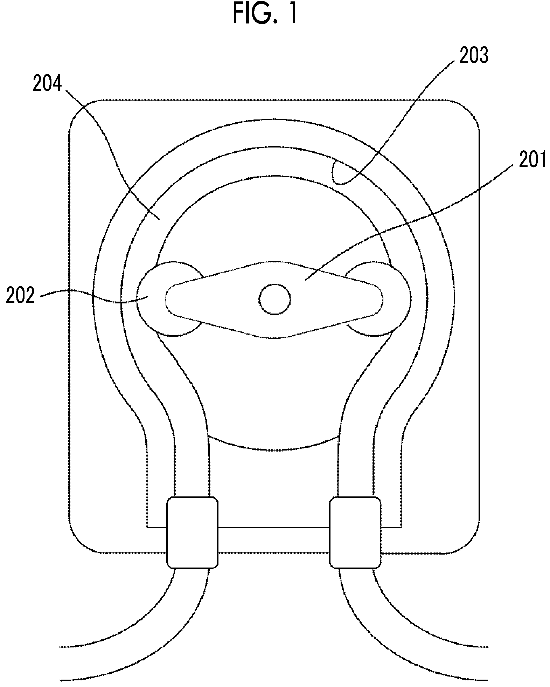

A tube pump is generally known as a device for feeding liquid. Fig. 1 is a diagram showing an example of a configuration of the tube pump. The tube pump 200 includes a rotating part 201 that includes a rotating shaft at a central portion thereof, rollers 202 that are attached to the periphery of the rotating part 201, and a flexible tube 204 that is attached to an outer wall 203 surrounding the rotating part 201 to have a portion to be in contact with the roller 202. The portion of the tube 204 that is in contact with the roller 202 is sandwiched between the roller 202 and the outer wall 203. As the rotating part 201 is rotated, the rollers 202 are rotated while crushing the tube 204. Accordingly, a negative pressure is generated on the suction side of the tube 204, so that liquid can be sucked into the tube. The liquid sucked into the tube is fed to the discharge side by the rotation operation of the rotating part 201. This operation is repeated, so that the liquid can be continuously fed.

In the case of the feeding of the liquid using the tube pump, the liquid to be fed is not in contact with a member other than the tube. Accordingly, it is possible to suppress a risk of contamination of the liquid. For this reason, the tube pump is particularly useful as, for example, a liquid feeding unit of a cell processing apparatus.

On the other hand, the tube pump has a disadvantage that pulsation occurs due to the principle of operation thereof. In the case of the feeding of the liquid using the tube pump, a flow velocity periodically fluctuates during the feeding of the liquid. The periodic fluctuation of the flow velocity is pulsation. In the case of applications in which liquid is required to be fed at a constant flow velocity, pulsation is a disadvantage.

The disclosed technology has been made in view of the above points, and an object of the disclosed technology is to suppress the pulsation of liquid flowing in a flow passage.

A liquid feeding assistance device according to an aspect of the disclosed technology comprises a tube that is expandable and contractible depending on a flow velocity of liquid flowing in the tube, and a gripping member that grips the tube with a relatively small force during expansion of the tube and grips the tube with a relatively large force during contraction of the tube.

The gripping member may grip the tube with a magnetic force. The gripping member may include a magnet and a magnetic body that faces each other with the tube interposed between the magnet and the magnetic body. The gripping member may include a pair of magnets of which polarities of surfaces facing each other with the tube interposed between the surfaces are different from each other.

The gripping member may be attachable to and detachable from the tube. The tube may be a single-use tube.

The gripping member may grip the tube such that an area of a cross section of a flow passage, which is formed by the tube, orthogonal to a flow direction of the liquid is smaller than an area of the cross section of the flow passage in a natural state.

A cell processing apparatus according to another aspect of the disclosed technology comprises a filter device that includes a hollow fiber membrane for filtering liquid, a tube pump that feeds the liquid to the filter device, and the liquid feeding assistance device that is provided on a flow passage between the filter device and the tube pump.

A liquid feeding assistance method according to still another aspect of the disclosed technology is a liquid feeding assistance method in a case where liquid is fed using a tube that is expandable and contractible depending on a flow velocity of the liquid flowing in the tube, and comprises gripping the tube with a relatively small force during expansion of the tube, and gripping the tube with a relatively large force during contraction of the tube.

According to the disclosed technology, it is possible to suppress the pulsation of liquid flowing in a flow passage.

### BRIEF DESCRIPTION OF THE DRAWINGS

Fig. 1 is a diagram showing an example of a configuration of a tube pump.
Fig. 2 is a perspective view showing an example of a configuration of a liquid feeding assistance device according to an embodiment of a disclosed technology.
Fig. 3 is a sectional view showing the example of the configuration of the liquid feeding assistance device according to the embodiment of the disclosed technology.
Fig. 4 is a cross-sectional view showing the example of the configuration of the liquid feeding assistance device according to the embodiment of the disclosed technology.
Fig. 5 is a diagram showing a gripping member in a state where the gripping member does not grip a tube according to the embodiment of the disclosed technology.
Fig. 6 is a diagram showing examples of a state change in a cross section of the tube in a case where liquid causing pulsation is made to flow in the tube.
Fig. 7 is a diagram showing an example of a configuration of a damper mechanism.
Fig. 8 is a diagram showing an example of a configuration of a liquid feeding assistance device according to another embodiment of the disclosed technology.
Fig. 9A is a diagram showing an example of a state of an orthogonal cross section of a tube in a case where liquid causing pulsation is made to flow in the tube gripped by a gripping member according to the embodiment of the disclosed technology.
Fig. 9B is a diagram showing an example of a state of the orthogonal cross section of the tube in a case where liquid causing pulsation is made to flow in the tube gripped by the gripping member according to the embodiment of the disclosed technology.
Fig. 10 is a diagram showing an example of a configuration of a liquid feeding assistance device according to another embodiment of the disclosed technology.
Fig. 11 is a diagram showing an example of a configuration of a cell processing apparatus according to another embodiment of the disclosed technology.
Fig. 12A is a diagram schematically showing an aspect of filtering performed using a hollow fiber membrane.
Fig. 12B is a diagram showing a state where a flow direction of liquid is substantially perpendicular to a membrane surface of the hollow fiber membrane.

### DESCRIPTION OF THE PREFERRED EMBODIMENTS

Hereinafter, an example of an embodiment of a disclosed technology will be described with reference to the accompanying drawings. The same or equivalent components and parts in the drawings will be denoted by the same reference numerals, and repeated description thereof will be omitted.

### First embodiment

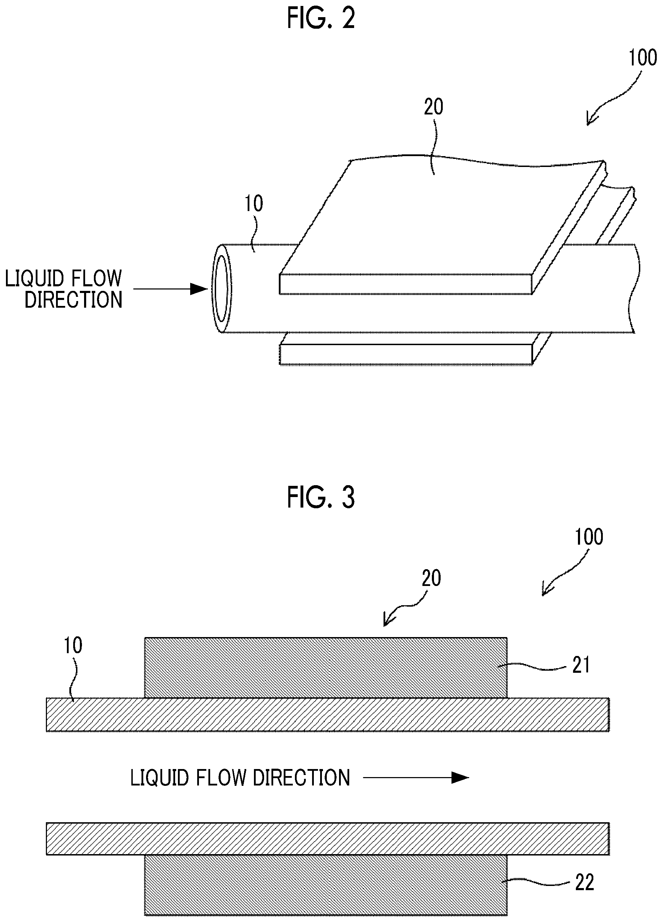

Fig. 2 is a perspective view showing an example of a configuration of a liquid feeding assistance device 100 according to a first embodiment of the disclosed technology. The liquid feeding assistance device 100 includes a tube 10 and a gripping member 20. The tube 10 is a member that forms a flow passage for liquid, and is a flexible tubular member that can expand and contract depending on a flow velocity of liquid flowing in the tube. The tube 10 may be, for example, a silicone tube. The tube 10 can be connected to a tube pump 200 shown in Fig. 1, and liquid fed from the tube pump 200 can flow in the tube 10. The liquid feeding assistance device 100 can be used as, for example, a component of a cell processing apparatus, and the liquid flowing in the tube 10 may be, for example, a cell suspension. In this case, the tube 10 may be a single-use (disposable) tube.

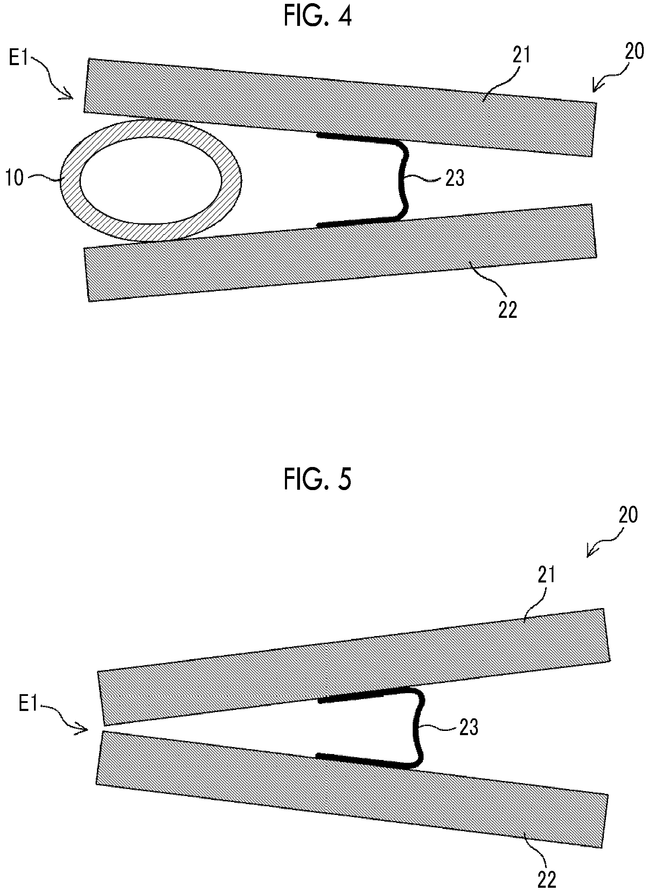

Figs. 3 and 4 are a sectional view and a cross-sectional view of the liquid feeding assistance device 100, respectively. Fig. 3 shows a cross section parallel to a flow direction of the liquid flowing in the tube 10 (hereinafter, referred to as a liquid flow direction), and Fig. 4 shows a cross section orthogonal to the liquid flow direction (hereinafter, referred to as an orthogonal cross section). The gripping member 20 grips the tube 10 such that an area of an orthogonal cross section of a flow passage formed by the tube 10 (hereinafter, referred to as an orthogonal cross-sectional area) is smaller than that in a natural state in a state where the liquid does not flow in the tube 10. The natural state is a state where no force acts on the tube 10 and the tube 10 neither expands nor contracts. The shape of the orthogonal cross section of the tube 10 in the natural state is a circular shape. In a case where the tube 10 is gripped by the gripping member 20, the tube 10 is brought into a crushed state and the orthogonal cross section of the tube 10 has an elliptical shape. As a result, the orthogonal cross-sectional area of the tube 10 is smaller than that in the natural state.

Fig. 5 is a diagram showing the gripping member 20 in a state where the gripping member 20 does not grip the tube 10. The gripping member 20 includes a pair of members, that is, a first member 21 and a second member 22 facing each other with the tube 10 interposed therebetween. A leaf spring 23 is provided between the first member 21 and the second member 22. A force applied in a closing direction acts on end portions E1 of the first member 21 and the second member 22 due to an elastic force of the leaf spring 23. The tube 10 is sandwiched between the first member 21 and the second member 22 at the end portions E1 of the first member 21 and the second member 22 as shown in Fig. 4, so that the tube 10 is gripped. That is, the gripping member 20 grips the tube 10 with the elastic force of the leaf spring 23. In a case where the tube 10 is gripped by the gripping member 20, the tube 10 is brought into a crushed state and the orthogonal cross-sectional area of the tube 10 is smaller than that in the natural state.

The gripping member 20 is attachable to and detachable from the tube 10. Accordingly, even in a case where the tube 10 is used as a single-use tube, the gripping member 20 can be repeatedly used. A torsion spring (torsion coil spring) can also be used instead of the leaf spring 23.

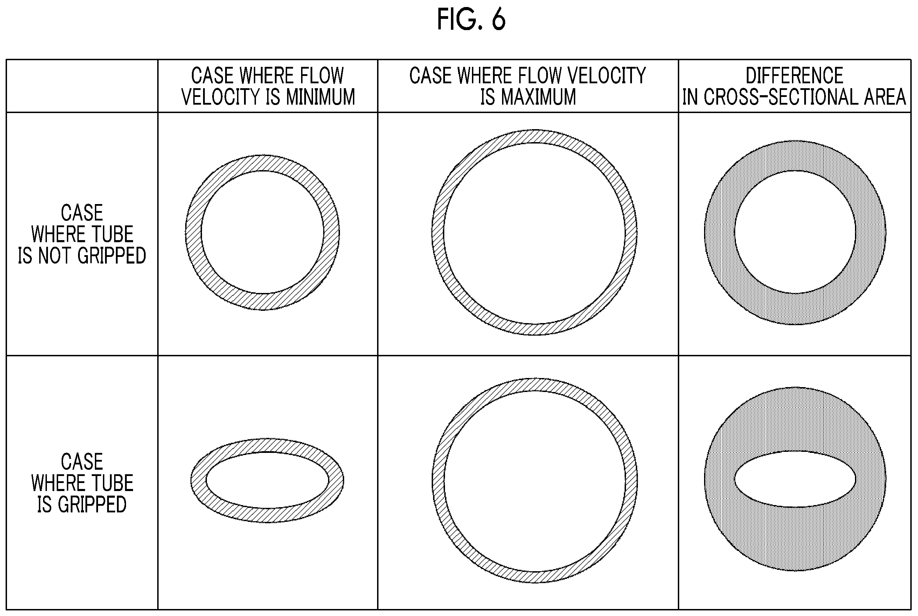

According to the liquid feeding assistance device 100 of the present embodiment, the pulsation of the liquid flowing in the tube 10 can be suppressed. The reason for this will be described below. Fig. 6 is a diagram showing examples of a state change in the orthogonal cross section of the tube 10 in a case where liquid causing pulsation is made to flow in the tube 10. An upper part in Fig. 6 shows a case where the tube 10 is not gripped, and a lower part in Fig. 6 shows a case where the tube 10 is gripped such that the orthogonal cross-sectional area of the tube 10 is smaller than that in the natural state.

In a case where the tube 10 is not gripped (the upper part in Fig. 6), the tube 10 is maintained in the natural state or is in a state where the tube 10 slightly contracts from the natural state at a timing when the flow velocity of the liquid flowing in the tube 10 is minimum. On the other hand, at a timing when the flow velocity of the liquid flowing in the tube 10 is maximum, the tube 10 expands due to the pressure of the liquid and the orthogonal cross-sectional area of the tube 10 is larger than that in the natural state.

In a case where the tube 10 is gripped such that the area of the cross section of the tube 10 orthogonal to the liquid flow direction is smaller than that in the natural state (the lower part in Fig. 6), the tube 10 is maintained in a crushed state at a timing when the flow velocity of the liquid flowing in the tube 10 is minimum. Accordingly, the orthogonal cross-sectional area of the tube 10 is smaller than that in the natural state. On the other hand, at a timing when the flow velocity of the liquid flowing in the tube 10 is maximum, the tube 10 expands due to the pressure of the liquid and the orthogonal cross-sectional area of the tube 10 is larger than that in the natural state.

On a right side in Fig. 6, a difference between the orthogonal cross-sectional area of the tube 10 at the maximum flow velocity and the orthogonal cross-sectional area of the tube 10 at the minimum flow velocity (hereinafter, referred to as a difference in cross-sectional area) is shown by a hatched region. As clear from the comparison between both the orthogonal cross-sectional areas, in a case where the tube 10 is gripped by the gripping member 20 such that the orthogonal cross-sectional area of the tube 10 is smaller than that in the natural state, a difference in cross-sectional area can be increased as compared to a case where the tube 10 is not gripped.

Since the tube 10 is expanded to hold the liquid in the tube 10 at a timing when the flow velocity is relatively high, the amount of liquid to be fed to the downstream side is suppressed. Since the tube 10 is contracted to promote the feeding of the liquid held in the tube 10 to the downstream side at a timing when the flow velocity is relatively low, pulsation is suppressed. That is, pulsation generated in the liquid flowing in the tube 10 is suppressed as the tube 10 expands and contracts. In a case where a difference in cross-sectional area (a difference between the orthogonal cross-sectional area of the tube 10 at the maximum flow velocity and the orthogonal cross-sectional area of the tube 10 at the minimum flow velocity) is increased, pulsation absorption ability, which is obtained through the expansion and contraction of the tube 10, can be enhanced.

According to the liquid feeding assistance device 100 of the embodiment of the disclosed technology, the gripping member 20 grips the tube 10 such that the orthogonal cross-sectional area of the tube 10 is smaller than that in the natural state. Accordingly, a difference in cross-sectional area can be increased. Therefore, since pulsation absorbing ability that is obtained through the expansion and contraction of the tube 10 can be increased, the pulsation of the liquid flowing in the tube 10 can be suppressed.

It is also conceivable to use a tube of which the shape of an orthogonal cross section in the natural state is an elliptical shape. However, in this case, an elastic force of the tube itself acts to inhibit the expansion of the tube while the tube expands from the natural state. On the other hand, according to the liquid feeding assistance device 100 of the embodiment of the disclosed technology, the tube 10 is brought into a crushed state from the natural state by the gripping member 20. Accordingly, while the tube 10 expands, the elastic force of the tube 10 itself acts to promote the expansion of the tube 10 until the tube 10 returns to the natural state. Therefore, according to the liquid feeding assistance device 100, an effect of suppressing pulsation can be enhanced as compared to a case where a tube of which the shape of an orthogonal cross section in the natural state is an elliptical shape is used.

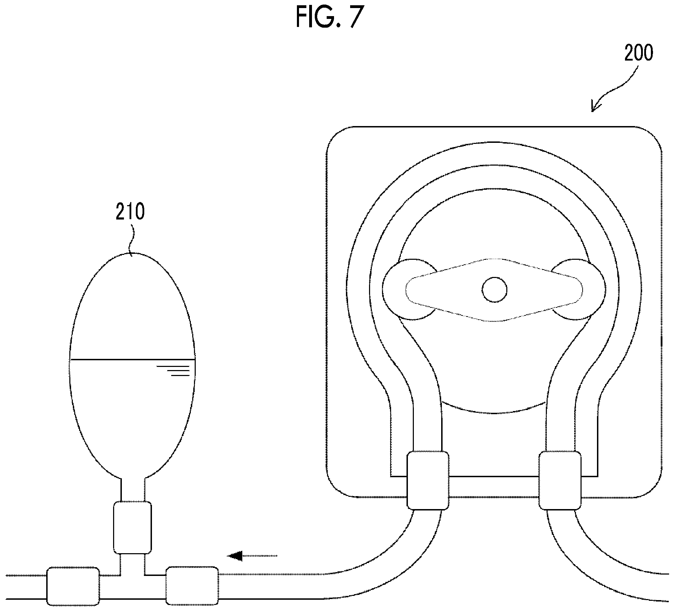

A method of connecting a damper mechanism 210, such as an air chamber or a tube damper, to a flow passage as shown in Fig. 7 is generally known as a method of suppressing the pulsation of liquid flowing through a flow passage. However, according to this method, the flow passage is complicated with the installation of the damper mechanism 210. Therefore, preparation is complicated. In particular, in a case where the flow passage is used as a single-use flow passage, the time and effort to replace the flow passage increases. For this reason, there is a concern that a working time and working errors may increase. Further, according to this method, a dead volume is increased. Therefore, this method is not suitable to feed expensive liquids, such as chemicals and regenerative medicine products.

On the other hand, according to the liquid feeding assistance device 100 of the embodiment of the disclosed technology, it is possible to obtain an effect of suppressing pulsation only by attaching the gripping member 20 to the tube 10. Further, since the gripping member 20 is attachable to and detachable from the tube 10 and is easily attached and detached, a working time and working errors can be minimized even in a case where the flow passage is used as a single-use flow passage. Furthermore, since the tube 10 itself forming the flow passage has a damper function, it is not necessary to separately provide a damper mechanism. Accordingly, the liquid feeding assistance device 100 can avoid the occurrence of a dead volume, and can be suitably used to feed expensive liquids such as chemicals and regenerative medicine products.

### Second embodiment

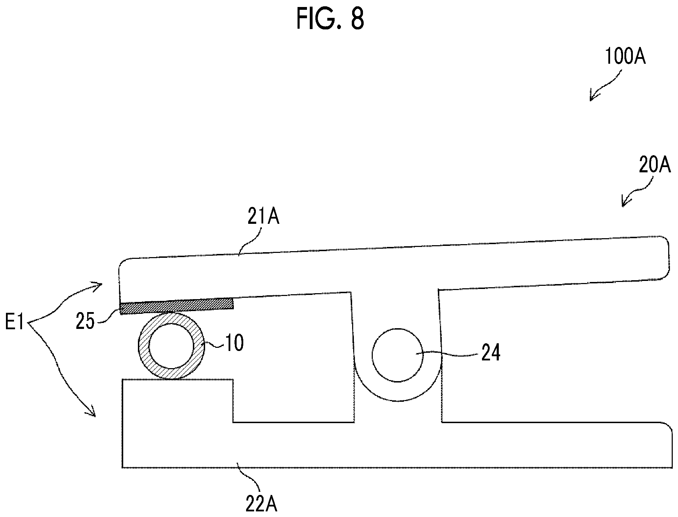

Fig. 8 is a diagram showing an example of a configuration of a liquid feeding assistance device 100A according to a second embodiment of the disclosed technology. The liquid feeding assistance device 100A includes a tube 10 and a gripping member 20A. The tube 10 is a member that forms a flow passage for liquid and is a flexible tubular member that can expand and contract depending on a flow velocity of liquid flowing in the tube as in the liquid feeding assistance device 100 according to the first embodiment.

The gripping member 20 according to the first embodiment described above grips the tube 10 with the elastic force of the leaf spring 23. The elastic force of the leaf spring 23 is relatively large during the expansion of the tube 10 and is relatively small during the contraction of the tube 10. Therefore, the gripping member 20 according to the first embodiment grips the tube 10 with a relatively large force during the expansion of the tube 10, and grips the tube 10 with a relatively small force during the contraction of the tube 10. On the other hand, the gripping member 20A according to the second embodiment grips the tube 10 with a relatively small force during the expansion of the tube 10, and grips the tube 10 with a relatively large force during the contraction of the tube 10. That is, in the gripping member 20A according to the second embodiment, an aspect of a change in a gripping force corresponding to the expansion and contraction of the tube 10 is opposite to that in the gripping member 20 according to the first embodiment.

The gripping member 20A includes a pair of members, that is, a first member 21A and a second member 22A facing each other with the tube 10 interposed therebetween. The first member 21A and the second member 22A are connected to each other at connecting portions 24 that are provided at middle portions of the first member 21A and the second member 22A in a longitudinal direction. The connecting portions 24 form a rotating shaft parallel to a flow direction of the liquid flowing in the tube 10, and the first member 21A and the second member 22A are rotationally movable with the connecting portions 24 as a fulcrum.

A magnet 25 is provided at an end portion E1 of the first member 21A, and an end portion E1 of the second member 22A is formed of a magnetic body such as iron. The entire second member 22A may be formed of a magnetic body. A force applied in a closing direction acts on the end portions E1 of the first member 21A and the second member 22A due to a magnetic force that is generated as the magnet 25 and the magnetic body come close to each other. The tube 10 is sandwiched between the first member 21A and the second member 22A at the end portions E1 of the first member 21A and the second member 22A, so that the tube 10 is gripped. That is, the gripping member 20A grips the tube 10 with the magnetic force of the magnet 25. Since the tube 10 is gripped with the magnetic force, a gripping force can be made relatively small during the expansion of the tube 10 and can be made relatively large during the contraction of the tube 10. As in the gripping member 20 according to the first embodiment, it is preferable that the gripping member 20A grips the tube 10 such that the orthogonal cross-sectional area of the flow passage formed by the tube 10 is smaller than that in the natural state in a state where liquid does not flow in the tube 10.

The gripping member 20A is attachable to and detachable from the tube 10. Accordingly, even in a case where the tube 10 is used as a single-use tube, the gripping member 20A can be repeatedly used.

As described above, since the tube 10 is expanded to hold the liquid in the tube 10 at a timing when the flow velocity is relatively high, the amount of liquid to be fed to the downstream side is suppressed. Since the tube 10 is contracted to promote the feeding of the liquid held in the tube 10 to the downstream side at a timing when the flow velocity is relatively low, the pulsation of the liquid flowing in the tube 10 is suppressed.

In a case where the tube 10 is gripped with the elastic force of a spring, a gripping force is increased as the tube 10 expands. Accordingly, a force that inhibits the expansion is increased as the tube 10 expands. Further, in a case where the tube 10 is gripped with the elastic force of a spring, a gripping force is reduced as the tube 10 contracts. Accordingly, it is difficult to crush the tube 10 to a state where the tube 10 contracts more than in the natural state. That is, in a case where the tube 10 is gripped with the elastic force of a spring, an effect of suppressing the pulsation of the liquid flowing in the tube 10 may be limited.

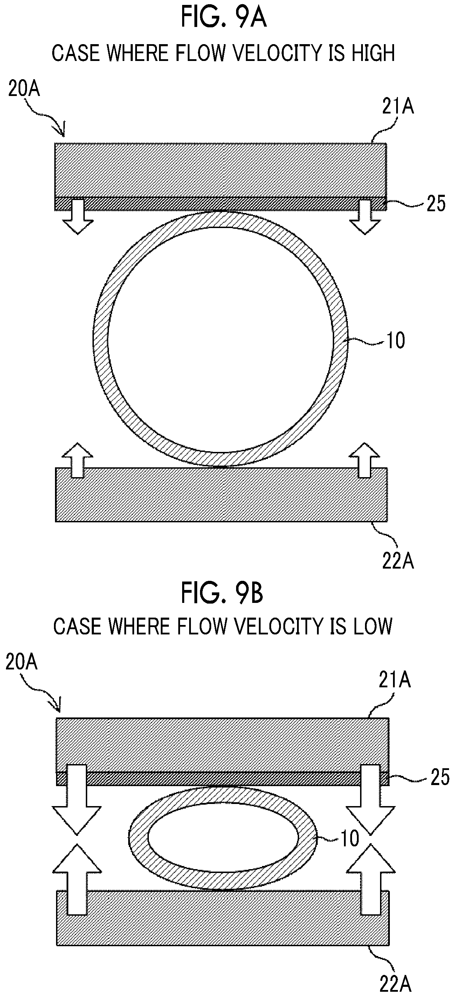

According to the liquid feeding assistance device 100A of the second embodiment of the disclosed technology, an effect of suppressing the pulsation of the liquid flowing in the tube 10 can be enhanced as compared to a case where the tube 10 is gripped with the elastic force of a spring. The reason for this will be described below. Figs. 9A and 9B are diagrams showing examples of the state of the orthogonal cross section of the tube 10 in a case where liquid causing pulsation is made to flow in the tube 10 gripped by the gripping member 20A, respectively. Fig. 9A shows a case where the flow velocity of the liquid flowing in the tube 10 is high, and Fig. 9B shows a case where the flow velocity of the liquid flowing in the tube 10 is low.

Since the tube 10 expands as shown in Fig. 9A at a timing when the flow velocity of the liquid flowing in the tube 10 is high, a distance between the first member 21A and the second member 22A of the gripping member 20A is increased. Accordingly, the magnetic force of the magnet 25 is reduced, so that a force with which the gripping member 20A grips the tube 10 is reduced. On the other hand, the distance between the first member 21A and the second member 22A of the gripping member 20A is reduced as shown in Fig. 9B at a timing when the flow velocity of the liquid flowing in the tube 10 is low. Accordingly, the magnetic force of the magnet 25 is increased, so that a force with which the gripping member 20A grips the tube 10 is increased.

As described above, according to the gripping member 20A that grips the tube 10 with a magnetic force, a force of gripping the tube 10 is reduced as the tube 10 expands. Therefore, a force that inhibits the expansion of the tube 10 is suppressed. Further, since the force of gripping the tube 10 is increased as the tube 10 contracts, the tube 10 can be crushed to a state where the tube 10 contracts more than in the natural state. Therefore, according to the liquid feeding assistance device 100A of the second embodiment, an effect of suppressing the pulsation of the liquid flowing in the tube 10 can be enhanced as compared to a case where the tube 10 is gripped with the elastic force of a spring.

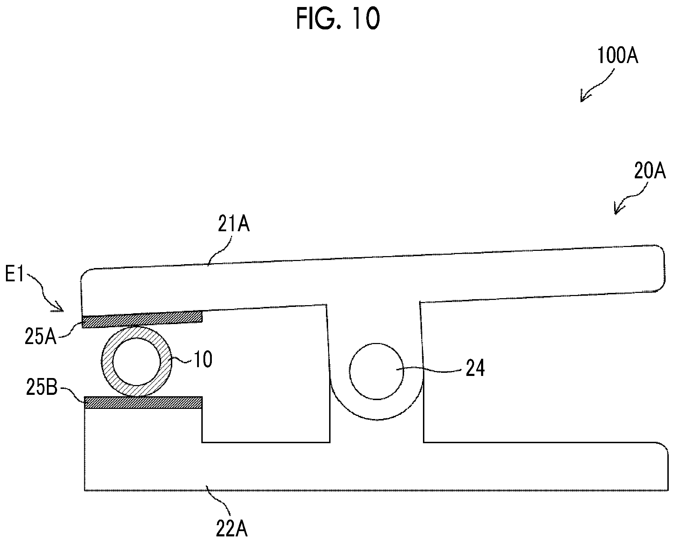

A case where the gripping member 20A includes the magnet 25 and the magnetic body that face each other with the tube 10 interposed therebetween has been exemplified in the above description, but the disclosed technology is not limited to this aspect. As shown in Fig. 10, the gripping member 20A may include a pair of magnets 25A and 25B of which polarities of surfaces facing each other with the tube 10 interposed therebetween are different from each other.

### Third embodiment

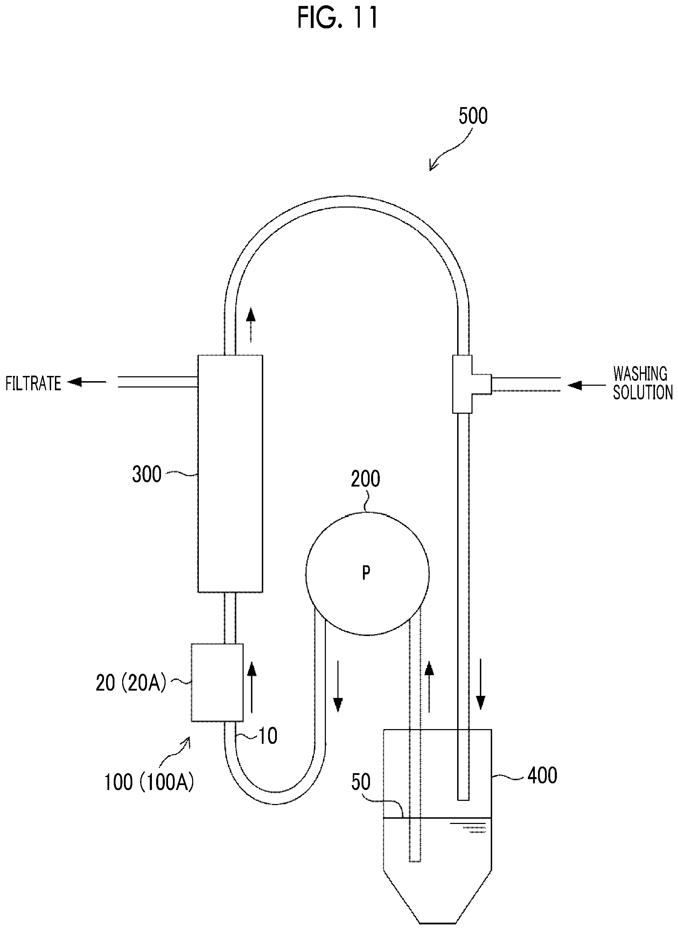

Fig. 11 is a diagram showing an example of a configuration of a cell processing apparatus 500 according to a third embodiment of the disclosed technology. The cell processing apparatus 500 may be used to manufacture, for example, regenerative medicine products. The regenerative medicine products are products that are made from the processing, such as culture, of living cells or tissues of a person or an animal, and are used for reconstructing, repairing, or forming a structure or function of a body, for treating or preventing a disease, or for being introduced into human cells for gene therapy. The regenerative medicine products include, for example, cultured skin and cultured cartilage.

The cell processing apparatus 500 according to the present embodiment performs washing treatment for cells, and includes the liquid feeding assistance device 100 or 100A described above. The cell processing apparatus 500 further includes a tube pump 200, a filter device 300, and a container 400.

Liquid 50 is stored in the container 400. The liquid 50 is a cell suspension. The tube pump 200 feeds the liquid 50, which is stored in the container 400, to the filter device 300. A flow passage for the liquid 50 is formed by a flexible tube 10. The liquid feeding assistance device 100 (100A) is provided on the flow passage between the tube pump 200 and the filter device 300.

The filter device 300 includes a hollow fiber membrane for filtering the liquid 50. Filtrate containing impurities, such as low-molecular-weight and protein components, which have permeated through the hollow fiber membrane, is discharged to the outside of a circulation system. The liquid 50 from which the impurities have been removed by the filtering performed by the filter device 300 is returned to the container 400. A flow passage for supplying a washing solution is connected to the flow passage between the filter device 300 and the container 400. The washing solution is supplied while the filtrate is discharged, so that the substitution of the liquid 50, that is, the washing of cells is performed.

In a case where the amount of the filtrate to be discharged per unit time and the amount of the washing solution to be supplied per unit time are equal to each other, the amount of the liquid flowing in the circulation system can be reduced to improve the substitution efficiency of the liquid. However, in a case where the amount of liquid flowing in the circulation system is small, the pulsation of the liquid to be generated due to the operation of the tube pump is more significant. In a system comprising a filter device including a hollow fiber membrane in a flow passage, an adverse effect caused by pulsation is more serious.

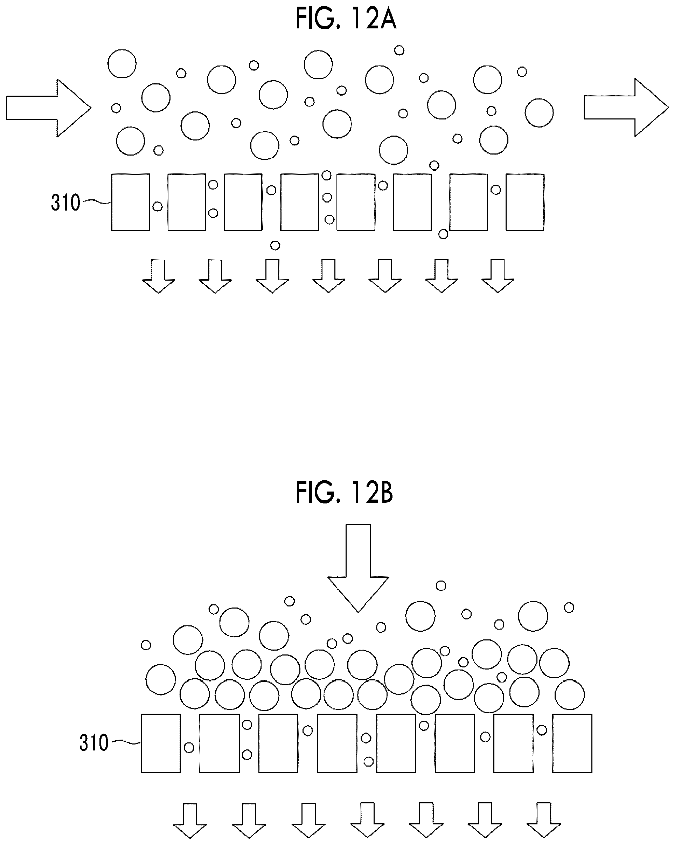

Fig. 12A is a diagram schematically showing an aspect of filtering performed using a hollow fiber membrane 310. The filtering performed using the hollow fiber membrane 310 is performed by a tangential flow filtration (TFF) method in which liquid to be treated flows along a membrane surface of the hollow fiber membrane 310. According to the TFF method, it is possible to continuously perform filtering while suppressing the blocking of the membrane surface of the hollow fiber membrane 310.

In a case where pulsation occurs in the liquid supplied to the hollow fiber membrane 310, the flow velocity of the liquid periodically becomes zero or close to zero. In a case where the flow velocity of the liquid supplied to the hollow fiber membrane 310 becomes close to zero, the filtration method is changed from the TFF method to a dead-end filtration method. In a case where the filtering performed using the hollow fiber membrane 310 is changed to the dead-end filtration method, the flow direction of the liquid is substantially perpendicular to the membrane surface of the hollow fiber membrane 310 as shown in Fig. 12B. As a result, the membrane surface of the hollow fiber membrane 310 is likely to be blocked (clogged). Therefore, in a system comprising a filter device including a hollow fiber membrane in a flow passage, it is particularly important to suppress the pulsation of the liquid flowing in the flow passage.

According to the cell processing apparatus 500 of the present embodiment, the liquid feeding assistance device 100 (100A) is provided on the flow passage between the filter device 300 including the hollow fiber membrane and the tube pump 200. Therefore, the liquid 50 of which pulsation is suppressed can be supplied to the filter device 300. It was confirmed that pulsation was suppressed in the liquid supplied to the filter device 300 by the liquid feeding assistance device 100 (100A).

According to the cell processing apparatus 500 of the present embodiment, the flow velocity of the liquid 50 supplied to the filter device 300 can be made constant. Therefore, it is possible to avoid the change of a method of filtering, which is performed by the filter device 300, to the dead-end filtration method. Accordingly, it is possible to suppress a risk of blocking (clogging) of the membrane surface of the hollow fiber membrane.

In regard to the first to third embodiments, the following supplementary notes will be further disclosed.

### Supplementary Note 1

A liquid feeding assistance device comprising:
a tube that is expandable and contractible depending on a flow velocity of liquid flowing in the tube; and
a gripping member that grips the tube with a relatively small force during expansion of the tube and grips the tube with a relatively large force during contraction of the tube.

Supplementary Note 2

The liquid feeding assistance device according to Supplementary Note 1,
in which the gripping member grips the tube with a magnetic force.

### Supplementary Note 3

The liquid feeding assistance device according to Supplementary Note 1 or 2,
in which the gripping member includes a magnet and a magnetic body that faces each other with the tube interposed between the magnet and the magnetic body.

### Supplementary Note 4

The liquid feeding assistance device according to Supplementary Note 1 or 2,
in which the gripping member includes a pair of magnets of which polarities of surfaces facing each other with the tube interposed between the surfaces are different from each other.

### Supplementary Note 5

The liquid feeding assistance device according to any one of Supplementary Notes 1 to 4,
in which the gripping member is attachable to and detachable from the tube.

### Supplementary Note 6

The liquid feeding assistance device according to any one of Supplementary Notes 1 to 5,
in which the tube is a single-use tube.

### Supplementary Note 7

The liquid feeding assistance device according to any one of Supplementary Notes 1 to 6,
in which the gripping member grips the tube such that an area of a cross section of a flow passage, which is formed by the tube, orthogonal to a flow direction of the liquid is smaller than an area of the cross section of the flow passage in a natural state.

### Supplementary Note 8

A cell processing apparatus comprising:
a filter device that includes a hollow fiber membrane for filtering liquid;
a tube pump that feeds the liquid to the filter device; and
the liquid feeding assistance device according to any one of Supplementary Notes 1 to 7 that is provided on a flow passage between the filter device and the tube pump.

### Supplementary Note 9

A liquid feeding assistance method in a case where liquid is fed using a tube that is expandable and contractible depending on a flow velocity of the liquid flowing in the tube, the method comprising:
gripping the tube with a relatively small force during expansion of the tube, and gripping the tube with a relatively large force during contraction of the tube.

### Explanation of References

10: tube
20, 20A: gripping member
21, 21A: first member
22, 22A: second member
24: connecting portion
25, 25A, 25B: magnet
50: liquid
100, 100A: liquid feeding assistance device
200: tube pump
201: rotating part
202: roller
203: outer wall
204: tube
210: damper mechanism
300: filter device
310: hollow fiber membrane
400: container
500: cell processing apparatus

## Claims

1. A liquid feeding assistance device comprising:
a tube that is expandable and contractible depending on a flow velocity of liquid flowing in the tube; and
a gripping member that grips the tube with a relatively small force during expansion of the tube and grips the tube with a relatively large force during contraction of the tube.

2. The liquid feeding assistance device according to claim 1,
wherein the gripping member grips the tube with a magnetic force.

3. The liquid feeding assistance device according to claim 2,
wherein the gripping member includes a magnet and a magnetic body that faces each other with the tube interposed between the magnet and the magnetic body.

4. The liquid feeding assistance device according to claim 2,
wherein the gripping member includes a pair of magnets of which polarities of surfaces facing each other with the tube interposed between the surfaces are different from each other.

5. The liquid feeding assistance device according to claim 1,
wherein the gripping member is attachable to and detachable from the tube.

6. The liquid feeding assistance device according to claim 1,
wherein the tube is a single-use tube.

7. The liquid feeding assistance device according to claim 1,
wherein the gripping member grips the tube such that an area of a cross section of a flow passage, which is formed by the tube, orthogonal to a flow direction of the liquid is smaller than an area of the cross section of the flow passage in a natural state.

8. A cell processing apparatus comprising:
a filter device that includes a hollow fiber membrane for filtering liquid;
a tube pump that feeds the liquid to the filter device; and
the liquid feeding assistance device according to any one of claims 1 to 7 that is provided on a flow passage between the filter device and the tube pump.

9. A liquid feeding assistance method in a case where liquid is fed using a tube that is expandable and contractible depending on a flow velocity of the liquid flowing in the tube, the method comprising:
gripping the tube with a relatively small force during expansion of the tube, and gripping the tube with a relatively large force during contraction of the tube.
